# EUROPEAN PATENT SPECIFICATION

(11) **EP 1 734 042 B1**
(45) Date of publication and mention of the grant of the patent: **09.09.2009**
(21) Application number: 06113454.0
(22) Date of filing: 03.05.2006
(51) Int. Cl.: C07D 501/00

(54) **Acid cefotetan totally solvent free and method for obtaining same**
Völlig lösungsmittelfreie Cefotetan Säure und Verfahren zu ihrer Herstellung.
Acide de Cefotetan complètement libre du solvant et un procédé pour sa préparation

(30) Priority: 10.06.2005 IT MI20051085
(43) Date of publication of application: 20.12.2006
(73) Proprietor: ACS DOBFAR S.p.A., 20067 Tribiano (Milano) (IT)
(72) Inventor: Zenoni, Maurizio, 20067 Paullo (MI) (IT); Donadelli, Alessandro, 26841Casalpusterlengo (LO) (IT)
(74) Representative: Frignoli, Luigi

(56) References cited:
- US-A- 4 263 432
- M. FUJIMOTO ET. AL.: "Process Development and Pilot Scale Synthesis of Cefotetan" ORGANIC PROCESS RESEARCH AND DEVELOPMENT, vol. 8, 2004, pages 915-919, XP002397340
- A. KOSHIRO ET. AL.: "Kinetics and Mechanism of Degradation and Tautomerization of Cefotetan in Aqueous Solution." CHEMICAL AND PHARMACEUTICAL BULLETIN, vol. 37, 1989, pages 1864-1869, XP001247578
- M. IWANAMI ET. AL.: "syntheiss of New Cephamycin Derivatives and a Novel Rearrangement Between Isothiazolethioacetamides and 1,3-Dithietanecarboxamides." CHEMICAL AND PHARMACEUTICAL BULLETIN, vol. 28, no. 9, 1980, pages 2629-2636, XP002209216

## Description

Cefotetan is an important antibiotic which has been used for some years in the therapy of infections from gram-negative bacteria resistant to the more common antibiotics, and is characterised by the formula

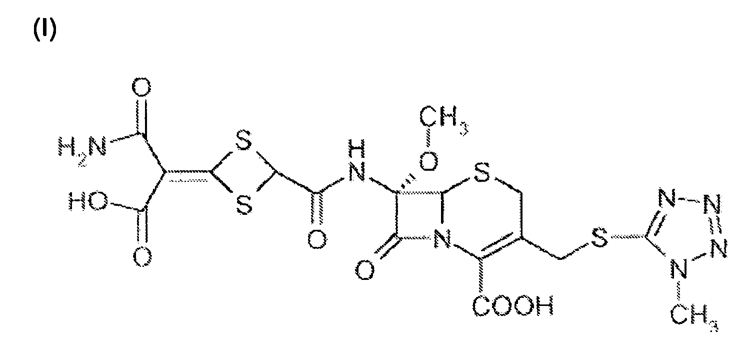

It is administered as the disodium salt by intravenous and intramuscular injection.

Cefotetan is claimed in US 4263432, claim 7 of which is specific for the compound of formula (I).

Claim 9 of that patent protects a tautomer thereof, which as the sodium salt is characterised by the formula

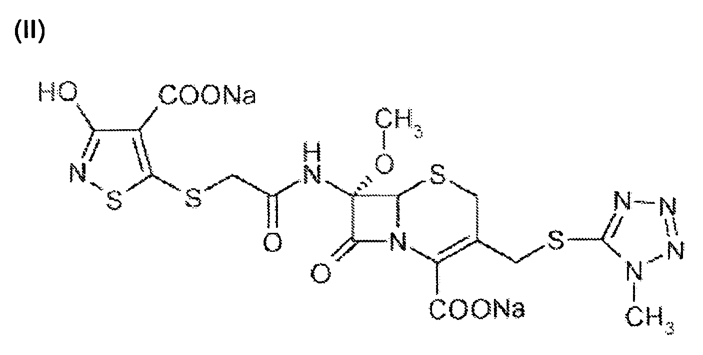

The product of formula (I) is obtained by a chemical synthesis process comprising numerous steps well known in the literature, however on termination of the process the product obtained contains a small percentage of the compound of formula (II) formed during the synthesis, its tautomerization to give the compound of formula (I) in the final solution not going to completion. Consequently, the injectable sterile solution contains the disodium salt of the compound of formula (I), together with a small quantity of the compound of formula (II). USA pharmacopea No. 28 defines a "cefotetan injection" as an isoosmotic sterile solution of the acid compound of formula (I) and of sodium bicarbonate in water for injections and one or more buffers. Analysis of cefotetan by liquid phase chromatography shows the presence of two peaks of different retention times, the solution containing not less than 90% and not more than 120% of the cefotetan declared on the label.

A study published in Chem. Pharm. Bull. 37(7) 1864-1869 (1989) shows that cefotetan tautomerization is facilitated in aqueous solution at alkaline pH. At the same time, the microbiological activity of the two tautomers in vitro appears almost equivalent, whereas their pharmacokinetic behaviour can differ, consequently the two tautomers can differ from the clinical behaviour viewpoint. The aforestated evidently demonstrates that the known art does not embrace a method suitable for nullifying or at least minimizing the content of tautomer of formula (II) in the injectable sterile cefotetan of formula (I). The importance of a method which enables cefotetan to be prepared without impurities and with a tautomer (II) content (HPLC) not greater than 0.5% is therefore evident.

In organic Process Research and Development, vol. 8, p. 915-9(2004) there is described a process for the preparation of Cefotetan containing 0.2% of tautomer, the process involving stepwise adjustment of the pH of the cefotetan aqueous solution with CO₂ from 8.5 to 7.5 followed by addition of HCl and also of methylethyl ketone as extraction solvent into which cefotetan is transferred from its initial solution. The methylethyl ketone phase containing cefotetan is separated from the aqueous phase, then it is concentrated and methanol is added to it to cause amorphous cefotetan to precipitate in the form of cotton wool particles containing also methylethyl ketone and methanol. The precipitate is first very slowly filtered into a centrifuge, then it is filtered again into a filter press to give a mass which is impregnated with (residual) methylethyl ketone and methanol and which is suspended in ethanol and filtered again in a filter press from which a mass can be recovered which contains undesirable amounts of the three above mentioned solvents which cannot be eliminated.

Moreover, the organic mother liquors (mainly consisting of a mixture of the three solvents) must be submitted to a separate recovery process "as the filtrate contained approximately 22% of 10 and 5% of 9", the number "10" meaning cefotetan.

It is obvious that the procedure described in the hereabove mentioned publication is very complex and leads to the production of cefotetan which is really free from impurities and of the tautomer, but which is rich of solvents which are unacceptable for the pharmaceutical use.

The inventors of the present application have surprisingly discovered a process by which the content of the tautomer of formula (II) can be drastically reduced by a process which does not use solvents, but only water, to hence provide solvent-free cefotetan acid free from which an injectable lyophilized product is obtained which is likewise solvent-free. This fact is particularly important considering that commercially available cefotetan acid has an overall solvent content between 1.0 and 1.5%, whereas the sodium salt obtained from it contains a total of about 0.16% of the following solvents: ethanol, methanol, methylethylketone and n-butanol.

The process of the invention enables cefotetan of formula (I) to be obtained containing up to 0.5% of the tautomer of formula (II) in its acid form with K.F up to 2.5%, dry content at least 99.0% and totally free of solvents.

The invention concerns also the acid cefotetan of formula (I) which is obtained by the process of the invention.

According to this process an aqueous solution of cefotetan at pH 7.5 containing carbon dioxide and up to 5% of the said tautomer of formula (II) is acidified with an acid chosen from the group consisting of dilute hydrochloric acid and dilute phosphoric acid, while maintaining the solution under agitation and under vacuum at a temperature between 20° and 35°C in order to remove the carbon dioxide to allow crystallization of the cefotetan of formula (I), which is filtered off and washed characterized in that the acidification of said aqueous solution of cefotetan is carried out slowly and gradually first to pH 3.4 and then to pH 1.5, and in that said crystallized cefotetan (I) is washed only with water to give a solution which is returned to a pH less than 7 and is then adsorbed on HP 20 SS resin and eluted with osmotized water to obtain a solution from which the aforesaid cefotetan of formula (I) totally free of solvents is precipitated by acidification to pH 1.5 with dilute hydrochloric acid.

The implementation of the process will be more apparent from the ensuing detailed description of practical embodiment thereof, given by way of non-limiting example.

### EXAMPLE

An aqueous solution containing crude cefotetan is prepared by a method known in the literature, from 20 g of benzhydryl 7β-[2-bromoacetamido]-7α-methoxy-3-(1-methyltetrazol-5-yl) thiomethyl-3-cephem-4-carboxylate. The final step results in a carbon dioxide-containing solution with a tautomer content ≤ 5% at pH 7.5. At this point the tautomerization to cefotetan does not proceed further and then product is then usually isolated.

However, in this specific case the solution is acidified to pH 5.15 with 15% HCl at 10-15°C, controlling carbon dioxide development. The solution is maintained under agitation under a vacuum of 22 mm Hg at 20°C and constant pH of 5.15. 1.67 g of 10% phosphoric acid is added to correct the pH to 4.5. The solution is again put under vacuum to eliminate further carbon dioxide to obtain a solution containing 12.94 g of cefotetan in 260 ml. It is decolorized with 2 g of decolorizing carbon, which is filtered off an washed with three portions of demineralized water for a total of 53 ml. The decolorized solution is at pH 5. The pH is corrected to 3.5 with 10% phosphoric acid, the temperature brought to 30-35°C and again agitated under vacuum at 31 mmHg. The operation is repeated correcting to a constant pH 3.2 with 10% phosphoric acid and agitating under vacuum. The pH is further lowered to 2.5 with 5% HCl at 30°C while agitating under vacuum. This procedure is continued, further lowering the pH to 1.5 with 5% HCl at 30°C under vacuum, for 30 min.

The solution is then cooled to +5°C and maintained under agitation for a further 30 min. The cefotetan crystallizes, is filtered off, washed with 1% HCl and then with demineralized water, both precooled to +5°C. 33 g of crude moist cefotetan are obtained with a tautomer (II) content (HPLC) of 2-10%.

The product is fed into 152 ml osmotized water at +5°C, and 4.46 g of sodium bicarbonate are added in portions while maintaining the pH ≤ 7. The solution obtained is brought to pH 5.5 with 5% HCl, maintaining it under vacuum to remove the residual carbon dioxide.

The solution contains 12.9 g of cefotetan with 3.8% of tautomer. The solution obtained is fed over 41 min through a 32 mm diameter column containing 240 ml of suitably conditioned HP 20 SS resin. Elution is carried out with osmotized water. 250 g of a rich fraction containing ≤ 0.2% tautomer are collected in about 60 min.

The solution obtained is acidified at 20°C to pH 3.4-3.5 with 10% phosphoric acid and then maintained under vacuum at 20°C for 30 min. The temperature is raised to 30-35°C until the foam disappears, then 5% HCl is added to pH 2.5. The solution is maintained under agitation at 30°C for one hour. On termination the pH is further lowered to 1.5 with 5% HCl. After 30 min at 30°C the solution is cooled to 5°C and agitated at that temperature for 2 hours.

The mixture is filtered, the product washed with 50ml 5% HCl and then with 100 ml of osmotized water, both precooled to 5°C. The product is dried at 45°-50°C until K.F ≤ 2.5%.
Yield: 12.33 g with titre (HPLC) as such of 97.4%, a tautomer (II) content (HPLC) less than 0.5% and with no residual solvents.

## Claims

1. A process for obtaining cefotetan of formula 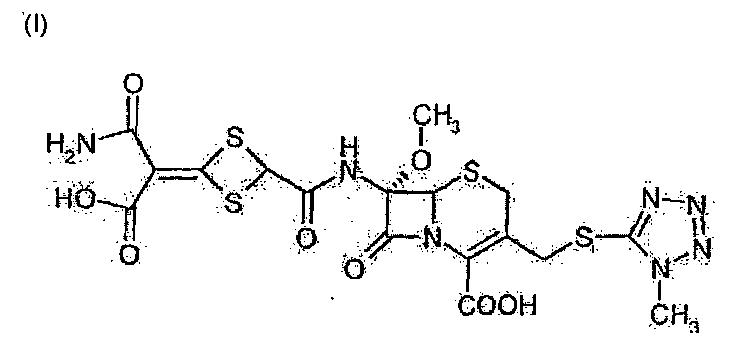 containing up to 0.5% of the tautomer of formula 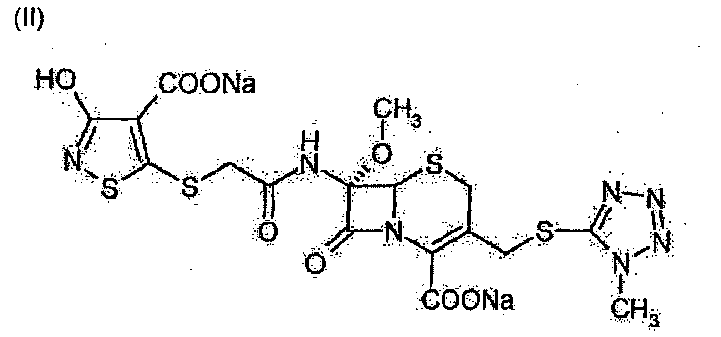 in its acid form with K.F. up to 2.5%, dry content at least 99.0% wherein an aqueous solution of cefotetan at pH 7.5 containing carbon dioxide and up to 5% of the said tautomer of formula (II) is acidified with an acid chosen from the group consisting of dilute hydrochloric acid and dilute phosphoric acid
while maintaining the solution under agitation and under vacuum at a temperature between 20° and 35°C in order to remove the carbon dioxide, to allow crystallization of the cefotetan of formula (I), which is filtered off and washed, **characterized in that** the acidification of said aqueous solution of cefotetan is carried out slowly and gradually first to pH 3.4 and then to pH 1.5, and **in that** said crystallized cefotetan (I) is washed only with water to give a solution which is returned to a pH less than 7 and is then adsorbed on HP 20 SS resin and eluted with osmotized water to obtain a solution from which the aforesaid cefotetan of formula (I) totally free of solvents is precipitated by acidification to pH 1.5 with dilute hydrochloric acid.

## Patentansprüche

1. Verfahren zur Herstellung von Cefotetan der Formel 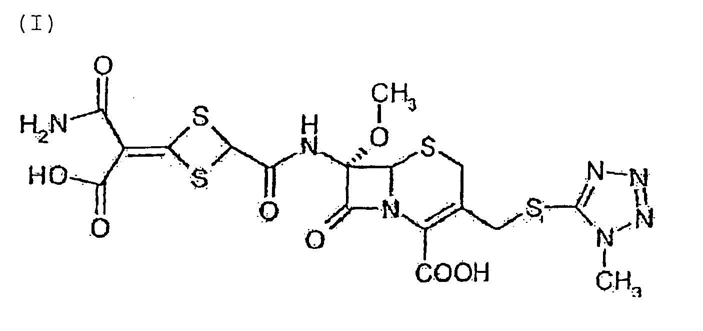 enthaltend bis zu 0,5% des Tautomers der Formel 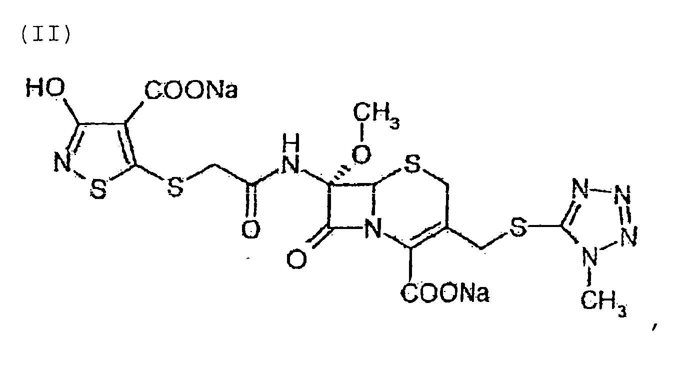 in seiner Säureform mit einem K.F.-Wert bis zu 2,5%, einem Trockengehalt von wenigstens 99,0%, wobei eine wässrige Lösung von Cefotetan mit pH 7,5, die Kohlendioxid und bis zu 5% des Tautomers der Formel (II) enthält, mit einer Säure, ausgewählt aus der Gruppe, bestehend aus verdünnter Salzsäure und verdünnter Phosphorsäure, acidifiziert wird, während die Lösung unter Rühren und unter Vakuum bei einer Temperatur zwischen 20° und 35°C gehalten wird, um das Kohlendioxid zu entfernen, um eine Kristallisation des Cefotetan der Formel (I) zuzulassen, welches abfiltriert und gewaschen wird, **dadurch gekennzeichnet , dass** die Acidifizierung der wässrigen Lösung von Cefotetan langsam und allmählich zuerst auf pH 3,4 und dann auf pH 1,5 durchgeführt wird und dass das kristallisierte Cefotetan (I) nur mit Wasser gewaschen wird, um eine Lösung zu ergeben, die auf einen pH von weniger als 7 zurückgebracht wird und dann an HP 20 SS-Harz adsorbiert wird und mit osmotisiertem Wasser eluiert wird, um eine Lösung zu erhalten, aus der das genannte Cefotetan der Formel (I) vollständig frei von Lösungsmitteln durch Acidifizierung auf pH 1,5 mit verdünnter Salzsäure präzipitiert wird.

## Revendications

1. Procédé destiné à obtenir du céfotétan de formule 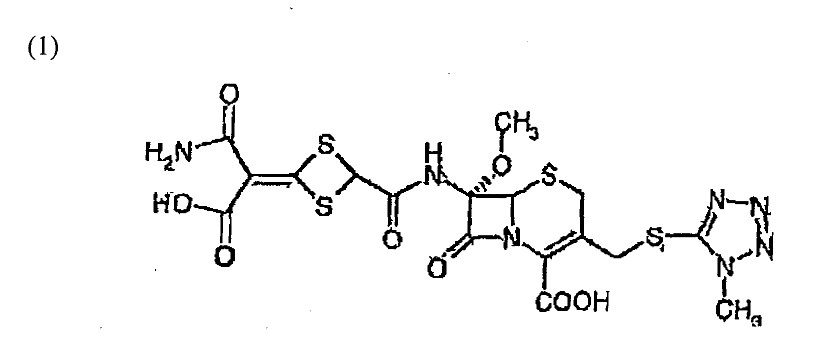 contenant jusqu'à 0,5 % du tautomère de formule 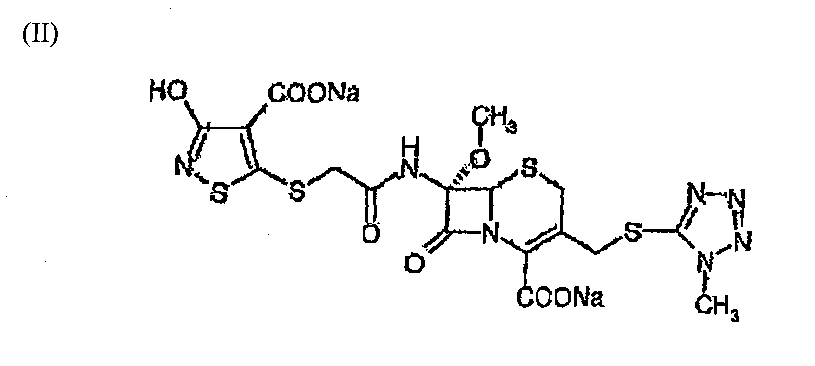 sous sa forme acide avec du K.F. jusqu'à 2,5 %, une teneur en matière sèche d'au moins 99,0 %, où une solution aqueuse de céfotétan à un pH de 7,5 contenant du dioxyde de carbone et jusqu'à 5 % dudit tautomère de formule (II) est acidifié avec un acide choisi dans le groupe constitué de l'acide chlorhydrique dilué et de l'acide phosphorique dilué tout en maintenant la solution sous agitation et sous vide à une température comprise entre 20 °C et 35 °C afin d'éliminer le dioxyde de carbone, pour permettre la cristallisation du céfotétan de formule (I), qui est éliminé par filtration et lavé, **caractérisé en ce que** l'acidification de ladite solution aqueuse de céfotétan est réalisée lentement et progressivement d'abord à un pH de 3,4, puis à un pH de 1,5, et **en ce que** ledit céfotétan cristallisé (I) est lavé uniquement avec de l'eau pour donner une solution qui est renvoyée à un pH inférieur à 7 et est ensuite adsorbée sur une résine HP 20 SS et éluée avec de l'eau osmotisée pour obtenir une solution à partir de laquelle le céfotétan précité de formule (I) totalement dépourvu de solvants est précipité par acidification à un pH de 1,5 avec de l'acide chlorhydrique dilué.
